# FASCICULE DE BREVET EUROPEEN

(11) **EP 3 986 748 B1**
(45) Date de publication et mention de la délivrance du brevet: **05.07.2023**
(21) Numéro de dépôt: 20743774.0
(22) Date de dépôt: 16.06.2020
(51) Int. Cl.: B60P 3/00, B60P 3/14, E04H 1/12, A61L 2/18

(54) **DISPOSITIF DE DECONTAMINATION APPROFONDIE DE PERSONNES**
VORRICHTUNG ZUR TIEFENKONTAMINATION VON PERSONEN
DEVICE FOR DEEP DECONTAMINATION OF PERSONS

(30) Priorité: 21.06.2019 FR 1906702
(43) Date de publication de la demande: 27.04.2022
(73) Titulaire: Utilis, 57365 Ennery (FR)
(72) Inventeur: PREVOST, Philippe, 57000 Metz (FR)
(74) Mandataire: Radzimski, Eric
(86) Numéro de dépôt international: PCT/IB2020/055607
(87) Numéro de publication internationale: WO 2020/254953

(56) Documents cités:
- US-A- 5 706 846
- US-A- 5 833 294
- US-B1- 8 650 806

## Description

### Domaine de l'invention

La présente invention concerne le domaine de la décontamination des personnes qui ont été soumises à des agents contaminants.

En particulier, la présente invention concerne un dispositif capable d'assurer la décontamination approfondie de personnes.

### Art antérieur

En cas d'accident industriel ou écologique, ou en cas d'attaque militaire ou terroriste impliquant une dispersion d'agents contaminants, notamment de type nucléaire, bactériologique ou chimique, il peut être nécessaire de décontaminer, dans les délais les plus brefs, les personnes ayant été mises en contact avec ces agents contaminants.

Une telle décontamination peut commencer par une décontamination primaire, ou décontamination d'urgence, qui est mise en oeuvre le plus rapidement possible après la contamination, au plus près du lieu de la contamination. Cette décontamination d'urgence a pour objectif de réduire la contamination sur les victimes, et de limiter au maximum le transfert d'agents contaminants pouvant propager la contamination.

Une décontamination secondaire, ou décontamination approfondie, peut être mise en oeuvre après la décontamination d'urgence. Elle vise à éliminer les agents contaminants encore présents sur la surface corporelle et pouvant être retirés sous l'action mécanique de l'eau. Elle implique la mise en oeuvre d'étapes précises, incluant un déshabillage destiné à détruire les vêtements contaminés, un nettoyage de décontamination, un rinçage, un séchage par tamponnement, suivi d'un contrôle de l'absence de contamination résiduelle. Ces différentes étapes doivent être réalisées en suivant un protocole particulier, qui ne peut être mis en oeuvre efficacement qu'avec un équipement spécialement conçu pour cette fonction.

Pour être efficace, de tels dispositifs de décontamination approfondie de personnes doivent être déployés au plus près de la zone de contamination, très rapidement après la survenue de celle-ci. Il est donc avantageux que ces dispositifs puissent être facilement transportables, pour pouvoir être déployés à l'emplacement voulu. Il apparaît cependant que, souvent, les dispositifs transportables nécessitent un temps d'installation relativement important avant d'être rendus opérationnels.
US5706846A divulgue par exemple un abri déployable d'un bâtiment comprenant un support fixé sur ledit bâtiment, un cadre articulé fixé de manière amovible au support et une tente fermée fixée audit cadre et audit panneau de façon à former un abri.
US8650806B1 divulgue quant à lui un abri déployable comprenant un panneau de plafond, un panneau de plancher et des panneaux de parois latérales, tous en matériau rigide.

Il existe donc un besoin d'un dispositif de décontamination de personnes qui puisse à la fois être transportable de façon efficace et mis en oeuvre dans un délai très réduit, par exemple après son transport.

### Objectifs de l'invention

La présente invention a pour objectif de pallier ces inconvénients de l'art antérieur.

En particulier, un des objectifs de l'invention est de proposer un dispositif de décontamination approfondie de personnes qui présente des dimensions le rendant apte à être facilement transporté.

Un autre objectif de l'invention est de proposer un tel dispositif de décontamination de personnes qui puisse être mis en oeuvre très rapidement, en cas de besoin.

Un autre objectif de l'invention est de proposer un tel dispositif de décontamination de personnes qui puisse être mis en oeuvre en n'exigeant que très peu de matériel ou de ressources extérieures au dispositif même.

Encore un autre objectif de l'invention est de proposer un tel dispositif de décontamination de personnes qui puisse être mis en oeuvre et exploité par un nombre restreint d'opérateurs.

### Exposé de l'invention

Ces objectifs, ainsi que d'autres qui apparaîtront plus clairement par la suite, sont atteints à l'aide d'un dispositif de décontamination approfondie de personnes qui comprend, selon l'invention, un conteneur comportant au moins une douche de décontamination, le conteneur présentant au moins un panneau, défini dans une ouverture d'une des parois verticales du conteneur, le panneau étant monté pivotant autour d'une charnière horizontale joignant un bord supérieur du panneau au conteneur, pour être mobile entre une première position, dans laquelle le panneau s'étend sensiblement verticalement et obture l'ouverture, une seconde position, dans laquelle le panneau s'étend sensiblement horizontalement et forme un plafond recouvrant une surface abritée, le dispositif de décontamination comprenant des parois aptes à clore la surface abritée, pour constituer au moins une pièce du dispositif de décontamination.

Dans la première position du panneau, qui est également appelée position fermée ou position verticale, le dispositif de décontamination est dans sa configuration fermée, dans laquelle il est compact et occupe un volume relativement réduit. Sa forme de conteneur lui permet d'être stocké et d'être transporté relativement facilement, en utilisant les moyens standards de stockage et de transport de conteneurs.

S'il est nécessaire de l'utiliser, le dispositif de décontamination peut être déployé très rapidement dans sa configuration déployée, le panneau pouvant passer dans sa seconde position, qui est également appelée position déployée ou position horizontale. Dans cette position, le panneau couvre un espace supplémentaire, formant une pièce qui est close par les parois, ce qui permet de réaliser la décontamination dans un volume plus important que le volume du conteneur contenant le dispositif de décontamination.

Avantageusement, les parois sont constituées par des toiles et fixées, par l'un de leur bords, à proximité d'au moins un des bords du panneau, de façon à se déployer par gravité quand le panneau est dans la seconde position, les toiles étant dimensionnées pour s'étendre depuis le panneau jusqu'au sol où est posé le conteneur, dans la seconde position.

De telles parois peuvent être rangées très facilement et se déployer rapidement, après le passage du panneau dans sa deuxième position.

Avantageusement, au moins une des toiles présente des moyens de fixation à un bord de l'ouverture du conteneur.

Avantageusement, les toiles sont fixées sur toute la longueur des bords du panneau qui ne portent pas la charnière.

Avantageusement, des vérins sont montés entre le conteneur et le panneau, aptes à participer au mouvement du panneau de sa première position vers sa seconde position.

De tels vérins rendent la manipulation du panneau très facile, et permet la mise en oeuvre rapide et facile du dispositif de décontamination.

Avantageusement, le dispositif de décontamination de personnes comprend des pieds aptes à s'étendre verticalement, quand le panneau est dans la seconde position, entre une portion du panneau éloignée du conteneur et le sol.

Avantageusement, au moins un des pieds est fixé au panneau par l'intermédiaire d'une liaison pivot.

Avantageusement, au moins un des pieds présente une longueur réglable.

Avantageusement, le dispositif de décontamination de personnes comprend des moyens d'éclairage fixés sur lesdits panneaux.

Avantageusement, le conteneur est équipé de roues.

Avantageusement, le conteneur présente deux panneaux, définis respectivement dans des ouvertures de deux parois verticales opposées du conteneur.

Avantageusement, le conteneur comprend au moins deux douches de décontamination alignées entre les ouvertures.

Avantageusement, le conteneur comprend des portes séparant les douches l'une de l'autre, et des portes séparant chacune des douches des surfaces abritées par les panneaux.

Avantageusement, le dispositif de décontamination de personnes comprend des cloisons amovibles aptes à former au moins deux couloirs, chacun des couloirs traversant le conteneur, chacune des ouvertures, et les espaces abrités par les panneaux, le conteneur comprenant au moins deux douches de décontamination alignées dans chacun des couloirs.

Avantageusement, le dispositif de décontamination de personnes comprend un dispositif de récupération de l'eau des douches.

### Liste des figures

L'invention sera mieux comprise à la lecture de la description suivante de modes de réalisation préférentiels, donnée à titre de simple exemple figuratif et non limitatif, et accompagnée des figures parmi lesquelles :
- [Fig.1] la figure 1 est une vue en perspective d'un dispositif de décontamination de personnes selon un mode de réalisation de l'invention, dans sa configuration fermée le rendant apte au transport ;
- [Fig.2] la figure 2 est une vue en perspective du dispositif de décontamination de la figure 1, lors de l'ouverture de l'un de ses panneaux mobiles ;
- [Fig.3] la figure 3 est une vue en perspective du dispositif de décontamination de la figure 1, dans lequel les panneaux mobiles sont placés en position horizontale ;
- [Fig.4] la figure 4 est une vue en perspective du dispositif de décontamination de la figure 1, dans une configuration déployée ;
- [Fig.5] la figure 5 est une vue en perspective écorchée du dispositif de décontamination de la figure 1 dans une configuration déployée, permettant de voir l'aménagement intérieur de ce dispositif ;
- [Fig.6] la figure 6 est une vue en perspective écorchée du dispositif de décontamination de la figure 1 dans une autre configuration déployée, permettant de voir l'aménagement intérieur de ce dispositif.

### Description détaillée de modes de réalisation de l'invention

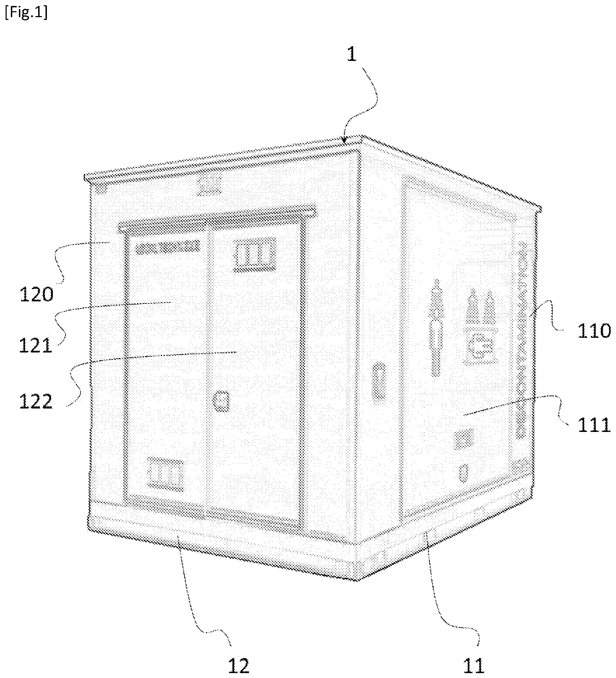

La figure 1 représente un conteneur 1 constituant un dispositif de décontamination approfondie de personnes, ou dispositif de décontamination de personnes, selon un mode de réalisation de l'invention. Ce conteneur 1 est un caisson métallique parallélépipédique dont les dimensions peuvent avantageusement être standardisées pour faciliter son transport, par exemple sur un camion.

Sur la figure 1, on voit l'une des faces latérales les plus longues du conteneur, appelée par la suite face avant 11 de ce conteneur. Cette face avant 11 est formée par un panneau fixe 110 définissant une ouverture dans laquelle est placé un panneau mobile 111. Le panneau fixe 110 entoure le panneau mobile 111, sur les côtés et au-dessus de celui-ci.

La figure 1 montre également l'une des faces latérales les plus courtes du conteneur 2 appelée par la suite face latérale gauche 12. Cette face latérale gauche 12 est constituée par un panneau fixe 120 définissant une ouverture dans laquelle sont placés des panneaux de portes 121 et 122. Le panneau fixe 120 entoure les panneaux de portes 121 et 122 sur les côtés et au-dessus de ces panneaux de portes.

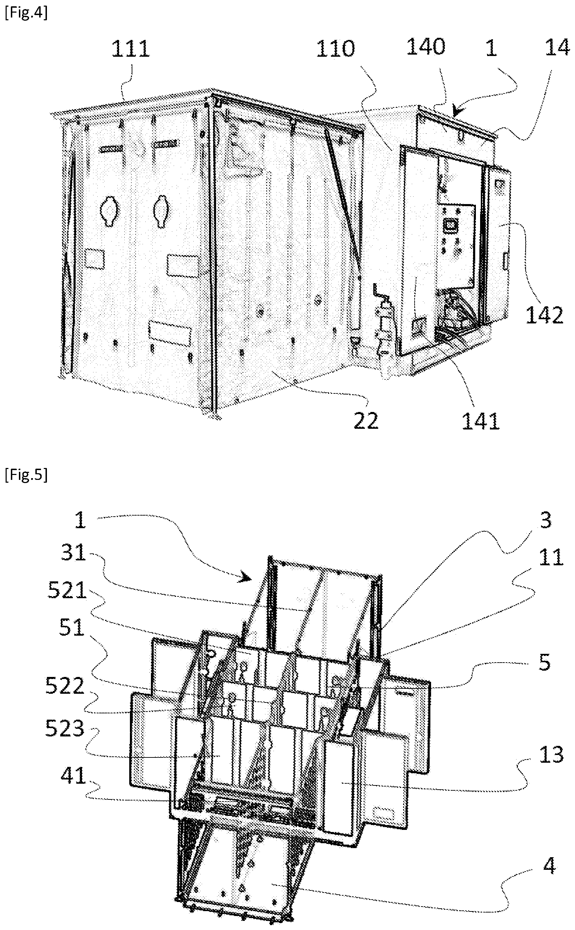

Le conteneur 1 présente également une face arrière 13, opposée à sa face avant 11, qui est visible notamment sur la figure 5. Cette face arrière est constituée d'un panneau fixe 130 définissant une ouverture dans laquelle est placé un panneau mobile 131, le panneau fixe et le panneau mobile étant disposés sensiblement comme sur la face avant 11.

Le conteneur 1 comporte enfin une face latérale droite 14, située à l'opposé de la face latérale gauche 12, et qui est constituée par un panneau fixe 140 définissant une ouverture dans laquelle sont placés des panneaux de portes 141 et 142, disposés sensiblement comme sur la face latérale gauche. Cette face latérale droite 14 est notamment visible sur la figure 4.

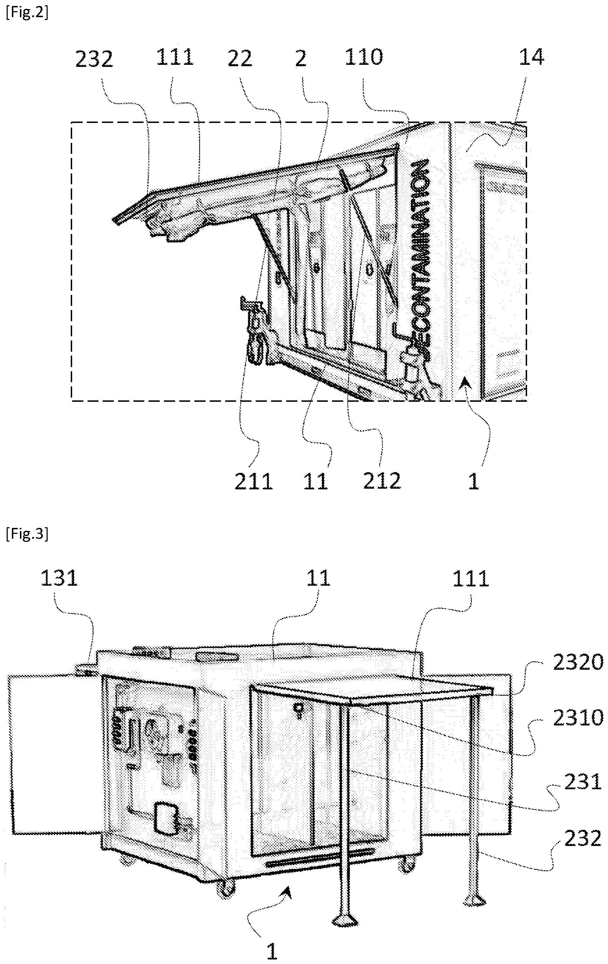

De façon avantageuse, le panneau mobile 111 peut pivoter autour d'une charnière horizontale, placée entre le bord supérieur horizontal du panneau mobile 111 et le panneau fixe 110. Le pivotement de ce panneau mobile 111 autour de cette charnière permet d'ouvrir la face avant du conteneur 1, comme le représente la figure 2.

Sur la figure 2, le panneau mobile 111 est représenté dans une position intermédiaire entre sa position fermée verticale, représentée sur la figure 1, dans laquelle elle obture l'ouverture de la face avant 11, et sa position déployée horizontale. On peut voir sur cette figure 2 que les bords latéraux du panneau mobile 111 sont reliés au panneau fixe 110 par des vérins à gaz 211 et 212. Ces vérins à gaz sont avantageusement dimensionnés pour assister au moins une partie du mouvement d'ouverture du panneau mobile 111 depuis sa position verticale représentée par la figure 1 jusqu'à sa position horizontale, qui est représentée par la figure 3.

La face interne 2 du panneau mobile 111, qui est à l'intérieur du conteneur 1 quand le panneau mobile 111 est fermé, porte avantageusement deux pieds pivotants et réglables 231 et 232 qui sont, dans la configuration représentée par la figure 2, repliés le long du bord du panneau mobile 111 opposé à la charnière.

Quand le panneau mobile 111 est placé en position horizontale, comme le représente la figure 3, ces deux pieds 231 et 232 peuvent pivoter chacun autour d'un pivot, respectivement 2310 et 2320, reliant une extrémité du pied, respectivement 231 et 232, à la face interne 2 du panneau mobile 111, à proximité des deux coins de ce panneau mobile 111 éloignés de la charnière. Quand le panneau mobile 111 est horizontal, les pieds 231 et 232 peuvent ainsi se placer dans une position verticale comme le représente la figure 3. La longueur de ces pieds 231 et 232 est par ailleurs réglable, de telle façon que ces pieds puissent prendre appui sur le sol pour maintenir le panneau mobile 111 dans sa position horizontale. Dans cette position représentée par la figure 3, le panneau mobile 111 forme un plafond, abritant la surface qu'il surplombe.

Par ailleurs, la face interne 2 porte également des toiles 22 enroulées qui sont fixées le long des trois bords du panneau mobile 111 qui ne portent pas la charnière. Avantageusement, un bord de chacune de ces toiles est fixé à la face interne 2.

Quand le panneau mobile 111 est dans sa position horizontale, les toiles 22 portées par sa face interne 2 peuvent être déroulées, l'un de leurs bords restant fixé à cette face interne 2. La figure 4 montre le conteneur 1 dont ces toiles 22 ont été déroulées. Ces toiles 22 s'étendent alors depuis le panneau mobile 111 jusqu'au sol. Elles peuvent être avantageusement assemblées entre elles et avec les bords du panneau fixe 110 de façon à enclore l'espace couvert par le panneau mobile 111.

Dans cette configuration représentée par la figure 4, l'espace couvert par le panneau mobile 111 forme ainsi une pièce close et couverte du dispositif de décontamination de personnes. De façon avantageuse, une ouverture peut être prévue dans l'une des toiles 24 formant cette pièce, par exemple sur la toile placée à l'opposé de l'ouverture du conteneur 1, afin de former une porte d'entrée dans cette pièce.

Dans le dispositif de décontamination de personnes selon le mode de réalisation représenté, cette pièce couverte par le panneau mobile 111 constitue un sas d'entrée 3. Dans un processus de décontamination, ce sas d'entrée 3 peut être utilisé pour le déshabillage des personnes contaminées, et le confinement des vêtements contaminés.

Avantageusement, un tapis de sol peut être placé sur le sol de ce sas d'entrée 3. Par ailleurs, la face interne 2 du panneau mobile 111 peut porter un éclairage, constitué par exemple par des lampes à LED, qui forment un plafonnier éclairant le sas de décontamination 3.

De façon avantageuse, le panneau mobile 131 de la face arrière 13 peut se déployer de la même manière que le panneau mobile 111, et est équipé de façon sensiblement identique afin de pouvoir abriter et clore une zone formant une pièce appelée par la suite sas de sortie 4. Dans un processus de décontamination, ce sas d'entrée peut être utilisé pour le séchage, le contrôle de l'absence d'agent contaminant sur les personnes ayant été décontaminées, et l'habillage avec des vêtements non contaminés.

La figure 5 est une vue en perspective écorchée du dispositif de décontamination quand le sas d'entrée 3 et le sas de sortie 4 sont installés, dans laquelle les plafonds ne sont pas représentés pour rendre visible l'aménagement intérieur de ce dispositif. À l'intérieur du conteneur 1, la zone située entre le sas d'entrée 3 et le sas de sortie 4 est appelée zone de nettoyage 5. Cette zone de nettoyage 5 est ouverte sur les sas d'entrée 3 et de sortie 4, les panneaux mobiles 110 et 131 laissant apparaître des ouvertures dans les faces avant 11 et arrière 13 quand ils sont dans leur position horizontale.

Le sas d'entrée 3, la zone de nettoyage 5 et le sas de sortie 4 sont, dans le mode de réalisation représenté par la figure 5, équipés de cloisons mobiles et de portes. Ainsi, des cloisons de séparation 31 dans le sas d'entrée 3, 51 dans la zone de nettoyage 5 et 41 dans le sas de sortie 4 séparent chacun de ces espaces en deux parties sensiblement égales. Ces cloisons de séparation permettent de définir, à travers le dispositif mobile de décontamination, deux couloirs de décontamination distincts, passant chacun, successivement, par le sas d'entrée 3, la zone de nettoyage 5 et le sas de sortie 4.

Ces cloisons de séparation peuvent par exemple être formées par des toiles, qui peuvent, dans le sas d'entrée 3 et le sas de sortie 4, être portées par les panneaux mobiles 111 et 131 et s'étendre jusqu'au sol pour cloisonner les espaces ou au contraire être enroulées pour supprimer le cloisonnement. Dans la zone de nettoyage 5, ces toiles peuvent être accrochées au plafond du conteneur 1 et/ou à des montants latéraux.

Dans la configuration représentée par la figure 5, la zone de nettoyage 5 comprend également des portes battantes séparant différents espaces. Dans chacun des deux couloirs de décontamination, des portes battantes 521 séparent ainsi le sas d'entrée 3 de la zone de nettoyage 5, des portes battantes 523 séparent la zone de nettoyage 5 du sas de sortie 4. Par ailleurs, des portes battantes 522 séparent la zone de nettoyage 5 en deux espaces.

Chacun de ces espaces de la zone de nettoyage 5 est équipé d'un pommeau de douche, pouvant être accroché à des supports à proximité du plafond. Dans un processus de décontamination, la douche située dans l'espace situé à proximité du sas d'entrée peut être utilisée pour le nettoyage de décontamination, et la douche dans l'espace située à proximité du sas de sortie peut être utilisée pour le rinçage.

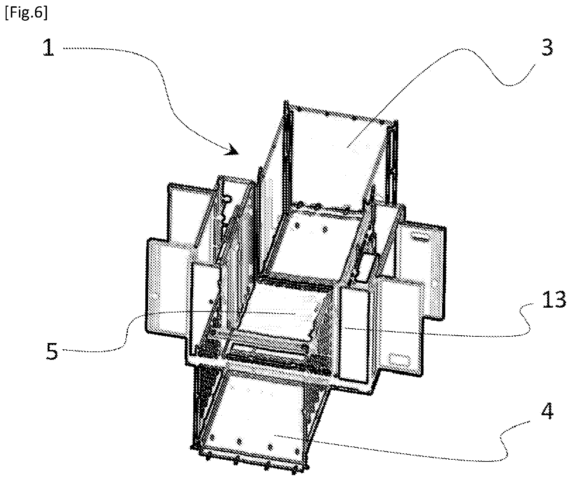

La figure 6 est une vue en perspective écorchée du dispositif de décontamination de la figure 1 dans une autre configuration déployée, permettant de voir l'aménagement intérieur de ce dispositif. Dans cette configuration, les cloisons de séparation ainsi que les portes battantes sont retirées, afin de définir dans le dispositif mobile de décontamination un unique circuit de décontamination de plus grande taille, permettant par exemple le passage d'une personne allongée sur un brancard, portée et décontaminée par des soignants.

Ce circuit unique de décontamination passe successivement par le sas d'entrée 3, la zone de nettoyage 5 et le sas de sortie 4. Dans cette configuration, les quatre pommeaux de douche de la zone de nettoyage 5 peuvent être décrochés de leurs supports pour être utilisés par les soignants afin de procéder à la décontamination de la personne allongée sur le brancard.

Les figures 5 et 6 montrent également, dans le conteneur 1, deux compartiments latéraux 61 et 62. Le compartiment 61, auquel il est possible d'accéder par les panneaux de portes 141 et 142 de la face latérale droite 14, est un compartiment de rangement. Le compartiment 62, auquel il est possible d'accéder par les panneaux de portes 121 et 122 de la face latérale gauche 12, contient les commandes des circuits hydrauliques, électriques et électroniques de fonctionnement du dispositif de décontamination.

Parmi les équipements hydrauliques contenus dans le dispositif de décontamination, il est avantageusement prévu un circuit d'alimentation en eau propre permettant l'alimentation des pommeaux de douche. Ce circuit peut comprendre, dans certains modes de réalisation, un système de chauffage de l'eau. Il peut avantageusement être branché au réseau d'alimentation en eau ou à une citerne mobile.

Il est également prévu, dans le plancher de la zone de nettoyage 5, un dispositif de collecte de l'eau usée, qui peut être connecté à un réservoir de collecte d'eau usée et potentiellement contaminée. Ce réservoir peut par exemple être un réservoir souple placé en dehors du conteneur 1.

Les équipements électriques et électroniques peuvent comprendre des moyens d'éclairage des différentes parties de l'équipement de décontamination et des systèmes de signalisation lumineuse visant à indiquer automatiquement aux utilisateurs, dans la configuration de la figure 5, le moment où ils doivent passer l'une des portes battantes, dans le cadre d'un processus de décontamination. Ce système de signalisation, associé à un système de détection de l'ouverture des portes battantes, permet au dispositif de réaliser la décontamination rapide de nombreuses personnes.

Le dispositif de décontamination de personnes peut également, en cas de besoin, contenir ou être associé à un équipement de chauffage par projection d'air chaud.

L'alimentation électrique de l'équipement de décontamination peut être faite par branchement à un réseau électrique ou à un équipement de production d'électricité, tel qu'un groupe électrogène.

Quand le dispositif de décontamination est replié, comme le représente la figure 1, il est entièrement contenu dans le conteneur 1 de forme parallélépipédique et peut être transporté facilement, par exemple sur un camion. Pour faciliter son transport, son embase peut prévoir des ouvertures destinées à introduire les lames de portage d'un transporteur de palettes. Le plafond du conteneur 1 peut également être équipé de crochets permettant son accrochage pour être soulevé par une grue. Enfin, le conteneur 1 peut également être équipé de roues, qui peuvent être amovibles, à proximité de ses quatre coins. De telles roues permettent de faciliter son transport sur des courtes distances.

## Revendications

1. Dispositif de décontamination approfondie de personnes, comprenant un conteneur (1) comportant au moins une douche de décontamination, ledit conteneur (1) présentant au moins un panneau (111), défini dans une ouverture d'une des parois verticales (11) dudit conteneur (1),
**caractérisé en ce que** ledit panneau (111) est monté pivotant autour d'une charnière horizontale joignant un bord supérieur dudit panneau (111) audit conteneur (1), pour être mobile entre :
- une première position, dans laquelle ledit panneau (111) s'étend sensiblement verticalement et obture ladite ouverture,
- une seconde position, dans laquelle ledit panneau (111) s'étend sensiblement horizontalement et forme un plafond recouvrant une surface abritée,
et **en ce que** ledit dispositif de décontamination comprend des parois aptes à clore ladite surface abritée, pour constituer au moins une pièce dudit dispositif de décontamination et **caractérisé en ce que** lesdites parois sont constituées par des toiles (22) et fixées, par l'un de leur bords, à proximité d'au moins un des bords dudit panneau (111), de façon à se déployer par gravité quand le panneau (111) est dans ladite seconde position, lesdites toiles (22) étant dimensionnées pour s'étendre depuis ledit panneau (111) jusqu'au sol où est posé le conteneur (1), dans ladite seconde position.

2. Dispositif de décontamination de personnes selon la revendication précédente, **caractérisé en ce qu'**au moins une desdites toiles (22) présente des moyens de fixation à un bord de ladite ouverture du conteneur (1).

3. Dispositif de décontamination de personnes selon l'une quelconque des revendications 1 et 2, **caractérisé en ce que** lesdites toiles (22) sont fixées sur toute la longueur des bords dudit panneau (111) qui ne portent pas ladite charnière.

4. Dispositif de décontamination de personnes selon l'une quelconque des revendications précédentes, **caractérisé en ce que** des vérins (211 et 212) sont montés entre ledit conteneur (1) et ledit panneau (111), aptes à participer au mouvement du panneau (111) de sa première position vers sa seconde position.

5. Dispositif de décontamination de personnes selon l'une quelconque des revendications précédentes, **caractérisé en ce qu'**il comprend des pieds (231, 232) aptes à s'étendre verticalement, quand ledit panneau (111) est dans ladite seconde position, entre une portion dudit panneau (111) éloignée dudit conteneur (1) et le sol.

6. Dispositif de décontamination de personnes selon la revendication précédente, **caractérisé en ce qu'**au moins un desdits pieds (231, 232) est fixé audit panneau (111) par l'intermédiaire d'une liaison pivot (2310, 2320).

7. Dispositif de décontamination de personnes selon l'une quelconque des revendications 5 et 6, **caractérisé en ce qu'**au moins un desdits pieds (231, 232) présente une longueur réglable.

8. Dispositif de décontamination de personnes selon l'une quelconque des revendications précédentes, **caractérisé en ce qu'**il comprend des moyens d'éclairage fixés sur lesdits panneaux.

9. Dispositif de décontamination de personnes selon l'une quelconque des revendications précédentes, **caractérisé en ce que** ledit conteneur (1) est équipé de roues.

10. Dispositif de décontamination de personnes selon l'une quelconque des revendications précédentes, **caractérisé en ce que** ledit conteneur (1) présente deux desdits panneaux (111, 131), définis respectivement dans des ouvertures de deux parois verticales (11, 13) opposées dudit conteneur (1).

## Patentansprüche

1. Vorrichtung zur Tiefen-Dekontamination von Personen, umfassend einen Container (1), der mindestens eine Dusche zur Dekontamination umfasst, wobei der Container (1) mindestens eine Platte (111) aufweist, die in einer Öffnung einer der vertikalen Wände (11) des Containers (1) definiert ist,
**dadurch gekennzeichnet, dass** die Platte (111) um ein horizontales Scharnier, das an einem oberen Rand der Platte (111) angebracht ist, schwenkbar am Container (1) montiert ist, um beweglich zu sein, zwischen:
- einer ersten Position, in der sich die Platte (111) im Wesentlichen vertikal erstreckt und die Öffnung verschließt,
- einer zweiten Position, in der sich die Platte (111) im Wesentlichen horizontal erstreckt, und eine Decke bildet, die eine geschützte Fläche abdeckt,
und dadurch, dass Vorrichtung zur Dekontamination Wände umfasst, die imstande sind, die geschützte Fläche einzuschließen, um mindestens einen Raum der Vorrichtung zur Dekontamination darzustellen, und **dadurch gekennzeichnet, dass** die Wände aus Tüchern (22) bestehen, und durch einen ihrer Ränder, in der Nähe von mindestens einem der Ränder der Platte (111) befestigt sind, um sich durch Schwerkraft zu entfalten, wenn die Platte (111) in der zweiten Position ist, wobei die Tücher (22) bemessen sind, um sich in der zweiten Position von der Platte (111) bis zum Boden zu erstrecken, wo der Container (1) aufgestellt ist.

2. Vorrichtung zur Dekontamination von Personen nach dem vorstehenden Anspruch, **dadurch gekennzeichnet, dass** mindestens eines der Tücher (22) Mittel zum Befestigen an einem Rand der Öffnung des Containers (1) aufweist.

3. Vorrichtung zur Dekontamination von Personen nach einem der Ansprüche 1 und 2, **dadurch gekennzeichnet, dass** die Tücher (22) über die gesamte Länge der Ränder der Platte (111) befestigt sind, die das Scharnier nicht tragen.

4. Vorrichtung zur Dekontamination von Personen nach einem der vorstehenden Ansprüche, **dadurch gekennzeichnet, dass** Zylinder (211 und 212) zwischen dem Container (1) und der Platte (111) angebracht sind, die imstande sind, sich an der Bewegung der Platte (111) von ihrer ersten Position in ihre zweite Position zu beteiligen.

5. Vorrichtung zur Dekontamination von Personen nach einem der vorstehenden Ansprüche, **dadurch gekennzeichnet, dass** sie Füße (231, 232) umfasst, die imstande sind, um sich, wenn die Platte (111) in der zweiten Position ist, vertikal zwischen einem Abschnitt der Platte (111), vom Container entfernt (1), und dem Boden zu erstrecken.

6. Vorrichtung zur Dekontamination von Personen nach dem vorstehenden Anspruch, **dadurch gekennzeichnet, dass** mindestens einer der Füße (231, 232) anhand einer Schwenkverbindung (2310, 2320) an der Platte (111) befestigt ist.

7. Vorrichtung zur Dekontamination von Personen nach einem der Ansprüche 5 und 6, **dadurch gekennzeichnet, dass** mindestens einer der Füße (231, 232) eine einstellbare Länge aufweist.

8. Vorrichtung zur Dekontamination von Personen nach einem der vorstehenden Ansprüche, **dadurch gekennzeichnet, dass** sie Beleuchtungsmittel umfasst, die an den Platten befestigt sind.

9. Vorrichtung zur Dekontamination von Personen nach einem der vorstehenden Ansprüche, **dadurch gekennzeichnet, dass** der Container (1) mit Rädern ausgestattet ist.

10. Vorrichtung zur Dekontamination von Personen nach einem der vorstehenden Ansprüche, **dadurch gekennzeichnet, dass** der Container (1) zwei Platten (111, 131) aufweist, die jeweils in Öffnungen von zwei gegenüberliegenden vertikalen Wänden (11, 13) des Containers (1) definiert sind.

## Claims

1. Device for deep decontamination of persons, comprising a container (1) having at least one decontamination shower, said container (1) having at least one panel (111), defined in an opening in one of the vertical walls (11) of said container (1),
**characterised in that** said panel (111) is mounted to pivot around a horizontal hinge which joins an upper edge of said panel (111) to said container (1) so as to be movable between:
- a first position, in which said panel (111) extends substantially vertically and closes off said opening,
- a second position, in which said panel (111) extends substantially horizontally and forms a ceiling covering a sheltered surface,
and **in that** said decontamination device comprises walls capable of closing off said sheltered surface, to form at least one portion of said decontamination device, and **characterised in that** said walls are formed by canvases (22) and fixed, by one of their edges, in the vicinity of at least one of the edges of said panel (111), so as to be deployed by gravity when the panel (111) is in said second position, said canvases (22) being sized to extend from said panel (111) to the ground where the container (1) is placed, in said second position.

2. Device for decontaminating persons according to the preceding claim, **characterised in that** at least one of said canvases (22) has means for fixing an edge of said opening of the container (1).

3. Device for decontaminating persons according to any one of claims 1 and 2, **characterised in that** said canvases (22) are fixed over the whole length of the edges of said panel (111) which do not carry said hinge.

4. Device for decontaminating persons according to any one of the preceding claims, **characterised in that** actuators (211 and 212) are mounted between said container (1) and said panel (111), capable of participating in the movement of the panel (111) from its first position to its second position.

5. Device for decontaminating persons according to any one of the preceding claims, **characterised in that** it comprises feet (231, 232) capable of extending vertically, when said panel (111) is in said second position, between a portion of said panel (111) remote from said container (1) and the ground.

6. Device for decontaminating persons according to the preceding claim, **characterised in that** at least one of said feet (231, 232) is fixed to said panel (111) by way of a pivot connection (2310, 2320).

7. Device for decontaminating persons according to any one of claims 5 and 6, **characterised in that** at least one of said feet (231, 232) has an adjustable length.

8. Device for decontaminating persons according to any one of the preceding claims, **characterised in that** it comprises lighting means fixed on said panels.

9. Device for decontaminating persons according to any one of the preceding claims, **characterised in that** said container (1) is equipped with wheels.

10. Device for decontaminating persons according to any one of the preceding claims, **characterised in that** said container (1) has two of said panels (111, 131), defined respectively in openings of two vertical walls (11, 13) opposite said container (1).
